**Europäisches Patentamt**

**European Patent Office**

**Office européen des brevets**

(19)

(11) Veröffentlichungsnummer: **0 253 965**
A1

(12)

# EUROPÄISCHE PATENTANMELDUNG

(21) Anmeldenummer: **87106193.3**

(22) Anmeldetag: **29.04.87**

(51) Int. Cl.³: **A 61 B 17/36**
**A 61 F 9/00**

(30) Priorität: **30.04.86 DE 8611912 U**

(43) Veröffentlichungstag der Anmeldung:
**27.01.88 Patentblatt 88/4**

(84) Benannte Vertragsstaaten:
**AT BE CH DE ES FR GB GR IT LI LU NL SE**

(71) Anmelder: **MEDITEC REINHARDT THYZEL GMBH**
**Obere Bergstrasse 3**
**D-8501 Heroldsberg(DE)**

(72) Erfinder: **Thyzel, Reinhardt**
**Obere Bergstrasse 3**
**D-8501 Heroldsberg(DE)**

(74) Vertreter: **Bischof, Hans-Jochen et al,**
**Schwalbenstrasse 10 Postfach 2105**
**D-2808 Syke-Barrien(DE)**

(54) **Vorrichtung zur Laserchirurgie an einem auf einem Operationstisch liegenden Patienten.**

(57) Vorrichtung zur Laserchirurgie an einem auf einem Operationstisch (1) liegenden Patienten (6) und insbesondere zur Laserbehandlung des Auges, mit einem Laser (2), einer Strahlführungseinrichtung (3), die den Laserstrahl auf die zu behandelnde Stelle führt, und einer Beobachtungseinrichtung (5) für die Operationsstelle, dadurch gekennzeichnet, daß der Laser (2) einschließlich der Steuerelektronik unter dem Operationstisch (1) angeordnet ist, und daß die Strahlführungseinrichtung (3) den Laserstrahl zunächst unter dem Operationstisch (1) in Richtung der Querachse des Operationstisches führt, im Randbereich des Operationstisches (1) senkrecht nach oben und oberhalb des Operationstisches in einen horizontalen Schwenkarm (32) umlenkt, an dessen strahlaustrittsseitigem Ende eine Umlenkeinrichtung (33) den Laserstrahl auf die Operationsstelle umlenkt, und dessen Länge verstellbar ist.

Fig. 2

EP 0 253 965 A1

# B e s c h r e i b u n g

Die Erfindung bezieht sich auf eine Vorrichtung zur Laserchirurgie an einem auf einem Operationstisch liegenden
Patienten und insbesondere auf eine Vorrichtung zur Laserbehandlung des Auges gemäß dem Oberbegriff des Anspruchs
1.

Die meisten bekannten Vorrichtungen zur Laserbehandlung
des Auges sind so aufgebaut, daß die zu behandelnde Person
sitzt. Während der Behandlung wird zwar der Kopf der zu
behandelnden Person von einer Kopf- und insbesondere einer
Kinnstütze gehalten, es ist aber unvermeidbar, daß Ermüdungserscheinungen bei der zu behandelnden Person während
längeren Behandlungen, wie sie bespielsweise bei der
radialen Keratotomie erforderlich sind, auftreten.

Deshalb ist vorgeschlagen worden, die zu behandelnde
Person während des Behandlungsvorgangs auf ein Bett bzw.
einen Operationstisch zu legen, so daß der Patient liegend
und damit wesentlich entspannter behandelt wird.

Bei einer Vorrichtung, von der zur Formulierung des Oberbegriffs des Anspruchs 1 ausgegangen worden ist, ist der
Laser neben dem Bett bzw. dem Operationstisch angeordnet.
Das Laserlicht wird mittels eines "Galgens" an die zu
behandelnde Stelle und insbesondere in das Auge geführt.
Die Beobachtungseinrichtung, beispielsweise ein Operationsmikroskop ist an dem Galgen befestigt.

Diese Vorrichtung gemäß dem Oberbegriff des Patentanspruchs 1 hat eine Reihe von Nachteilen:

Der Laser, der typischerweise Abmessungen von
1,5 m x 0,5 m x 1 m hat, ist in einem geringen Abstand vom

Operationstisch angeordnet, so daß die Bewegungsfreiheit des Arztes rund um den Operationstisch beschränkt ist. Darüberhinaus erlaubt die Befestigung des Operationsmikroskops am Galgen nicht die bei bestimmten Anwendungsfällen gewünschte Bewegungsfreiheit.

Vor allem aber gewährleistet die Art der Lichtführung mittels eines Galgens nicht die gewünschte Freiheit bei der Verstellung des Operations-Laserstrahls.

Der Erfindung liegt die Aufgabe zugrunde, eine Vorrichtung zu Laserchirurgie an einem auf einem Operationstisch liegenden Patienten und insbesondere zur Laserbehandlung des Auges anzugeben, bei der der Laserstrahl ohne Behinderung des Operateurs und mit den nötigen Einstellmöglichkeiten an die zu behandelnde Stelle geführt wird.

Eine erfindungsgemäße Lösung dieser Aufgabe ist mit ihren Weiterbildungen in den Ansprüchen gekennzeichnet. Erfindungsgemäß wird die Aufgabe ausgehend von einer Vorrichtung zur Laserchirurgie gemäß dem Oberbegriff des Anspruchs 1 dadurch gelöst, daß der Laser unter dem Operationstisch und die Strahlführungseinrichtung im Bereich der Bettkante angeordnet ist. Hierdurch wird erreicht, daß der Laser, dessen Abmessungen - wie bereits ausgeführt - in der Größenordnung von 1,5 x 0,5 x 1 m sein können, nicht die Bewegungsfreiheit bzw. den verfügbaren Raum im Behandlungszimmer beschränkt. Da die Strahlführungseinrichtung im wesentlichen ein senkrechtes Führungselement im Bereich der Kante des Operationstisches aufweist, das bei einem bevorzugten Ausführungsbeispiel an der Längsseite des Operationstisches (Anspruch 3) und insbesondere bei zur Laserbehandlung des Auges etwa in Brusthöhe des Patienten verläuft (Anspruch 4), wird die Bewegungsfreiheit des Operateurs sowie etwaiger Hilfspersonen praktisch nicht eingeschränkt. Die Verwendung eines Schwenkarms, aus dessen einem Ende der Laserstrahl austritt und dessen Länge verstellbar ist, gewährleistet eine praktisch beliebige Verstellmöglichkeit des Auftrefforts des Laserstrahls. Darüberhinaus können in dem Schwenkarm (Anspruch 5) nahezu beliebige Ausführungen von sogenannten Mikromanipulatoren angeordnet werden, mit denen beispielsweise durch Verkippung eines Spiegels der Auftreffort des Laserstrahls präzise verstellbar ist.

Weiterbildungen der Erfindung sind in den Unteransprüchen angegeben.

Selbstverständlich ist es möglich, den Operationstisch in üblicher Weise höhenverstellbar und/oder kippbar auszubilden. In jedem Falle ist es jedoch vorteilhaft, wenn der Schwenkarm, aus dem das Laserlicht austritt, zusätzlich höhenverstellbar ist, so daß leicht die Entfernung zwischen Austrittsort des Laserlichts und Auftreffort eingestellt werden kann.

Die erfindungsgemäße Art der Strahlführung erlaubt problemlos beispielsweise bei Operationen am Auge die Anordnung einer Spaltleuchte. Beispielsweise kann - wie im Anspruch 5 beansprucht - die Spaltleuchte im Schwenkarm angeordnet und das Laserlicht durch die Spaltleuchte geführt werden. Selbstverständlich ist es aber auch möglich, eine um eine zusätzliche oder gleiche Achse wie der Schwenkarm schwenkbare Spaltleuchte vorzusehen.

In jedem Falle ist es jedoch vorteilhaft, wenn die Beobachtungseinrichtung an einem weiteren Schwenkarm angebracht ist. Dies ermöglicht einen unabhängigen Freiheitsgrad bei der Einstellung der Beobachtungseinrichtung, so daß beispielsweise der Operationserfolg unter einem bestimmten Winkel zur Einstrahlrichtung des Laserlichts
beobachtet werden kann (Anspruch 6).

Die in den Ansprüchen 7 bis 10 gekennzeichneten Ausbildungen des weiteren Schwenkarms, an dem die Beobachtungseinrichtung angebracht ist, erhöhen die Einstellmöglichkeiten der Beobachtungseinrichtung, die insbesondere ein
Operationsmikroskop (Anspruch 11) sein kann, weiter.

Die im Anspruch 12 gekennzeichnete Ausbildung des Operationstisches trägt weiter zu einer guten Zugänglichkeit
des Behandlungsortes bei.

Die Erfindung wird nachstehend anhand eines Ausführungsbeispiels unter Bezugnahme auf die Zeichnung näher beschrieben, auf die ausdrücklich hinsichtlich der Offenbarung aller im Text nicht erläuterten Einzelheiten verwiesen wird. Es zeigen:

Fig. 1 eine Seitenansicht einer erfindungsgemäßen
        Vorrichtung, und

Fig. 2 eine Draufsicht auf die in Fig. 1 gezeigte
        Vorrichtung.

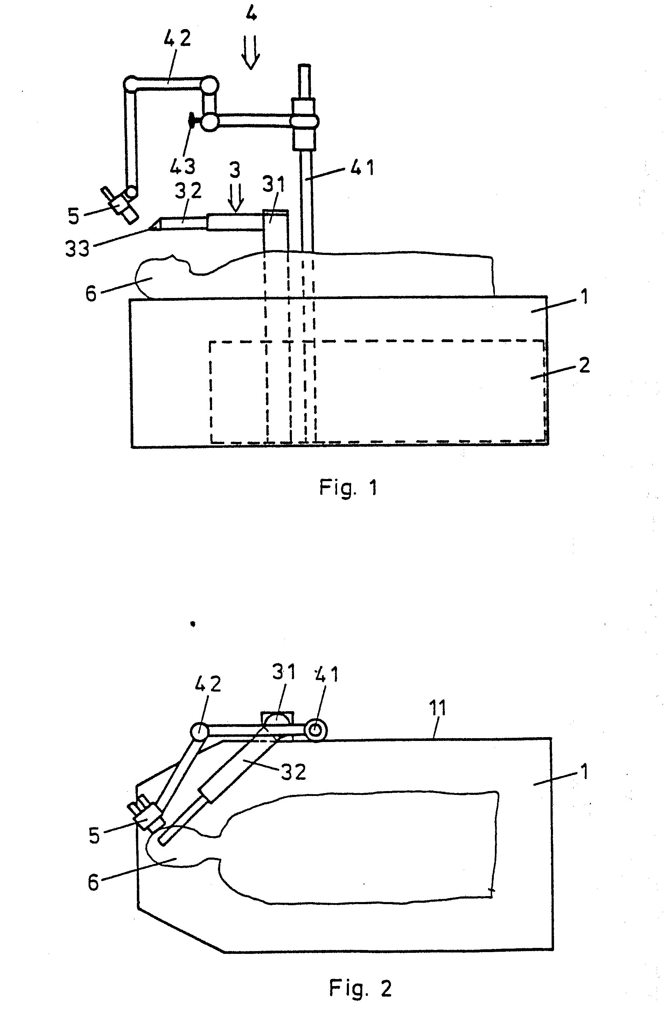

Das in den Fig. 1 und 2 dargestellte Ausführungsbeispiel
weist einen unter einem Operationstisch 1 angeordneten
Laser 2 auf. Hierbei wird unter Laser die Laserröhre
einschließlich sämtlicher zum Betrieb erforderlichen
Einheiten, wie Hochspannungsversorgung etc., verstanden.
Die Bedienungselemente des Lasers, wie beispielsweise die
Vorwahlelemente für die Leistung, Schußzeit etc., können
selbstverständlich an anderer Stelle, beispielsweise im
Bereich oder an einer der nachstehend beschriebenen Anordnungen vorgesehen sein. Ferner ist eine Strahlführungseinrichtung 3 vorgesehen, die den Laserstrahl des Lasers 2 an
den Behandlungsort, beispielsweise das Auge eines auf dem
Operationstisch 1 liegenden Patienten 6 lenkt. Die Strahlführungseinrichtung 3 besteht aus einer unmittelbar neben
einer Bett-Längskante 11 angeordneten senkrechten Säule 31
und einem Schwenkarm 32, der um eine durch die Säule 31
gehende Schwenkachse drehbar und dessen Länge mittels
eines "Teleskopauszuges" einstellbar ist. Der Schwenkarm
32 trägt an seinem vorderen Ende ein Prisma 33. Ferner ist
eine Halterung 4 für ein Operationsmikroskop 5 vorgesehen.
Die Halterung 4 besteht aus einer senkrechten Säule 41,
die ebenfalls unmittelbar neben der Bett-Längskante 11
angeordnet ist, einem mehrfach gekröpften Schwenkarm 42,
der um eine durch die Säule 41 gehende Drehachse schwenkbar und mittels einer Einstellschraube 43 zusätzlich um
eine horizontale Drehachse drehbar ist.

Die beschriebene Vorrichtung arbeitet wie folgt:

Der Laserstrahl des Lasers 2 verläuft zunächst unterhalb
des Operationstisches 1 horizontal und senkrecht zur
Längskante 11. Beim Eintritt in die senkrechte Säule 31
wird er um 90° nach oben umgelenkt und in dieser zum
horizontalen Schwenkarm 32 geführt. Durch eine nicht im
einzelnen dargestellte Umlenkeinrichtung wird der Laserstrahl dann in den Schwenkarm 32 umgelenkt und tritt aus
diesem nach einer nochmaligen Umlenkung durch das Prisma
33 in Richtung auf den Operationsort, also beispielsweise
das Auge des Patienten 6 aus. Durch die Schwenkbarkeit des
Schwenkarms 32 sowie die zusätzliche Einstellmöglichkeit
seiner Länge kann der Laserstrahl genau an die gewünschte
Stelle geführt werden. Zusätzlich ist es beispielsweise
möglich, einen der in der Strahlführungseinrichtung 3
angeordneten Spiegel bzw. eines der Prismen mittels einer
sogenannten Mikromanipulatoreinrichtung geringfügig zu kippen, um kleine Einstellbewegungen des Laserstrahls
durchzuführen.

Zur Kontrolle der Laserbehandlung dient das an der Einrichtung 4 angebrachte Operationsmikroskop 5. Dieses
Operationsmikroskop 5 ist nicht nur um eine durch die
Säule 41 gehende vertikale Drehachse schwenkbar, sondern
auch zusätzlich durch Verschieben des weiteren Schwenkarms
42 längs der Säule 41 höhenverstellbar. Ferner kann nach
Lösen der Einstellschraube 43 ein Teil des Schwenkarms 42
um eine im wesentlichen horizontale Achse verdreht, und
zusätzlich auch noch um weitere senkrechte (und andere)
Achsen in sich geschwenkt werden, so daß das Operationsmikroskop in eine nahezu beliebige Lage gebracht werden
kann.

Die erfindungsgemäße Vorrichtung hat damit den Vorteil,
daß die im Bereich der Längskante 11 angeordneten Strahlführungs- und Aufhängungseinrichtungen für das Operationsmikroskop die Bewegungsfreiheit des Operateurs sowie
etwaigem zusätzlichen Hilfspersonals praktisch nicht
behindern. Dabei ist es besonders vorteilhaft, wenn beispielsweise bei Operationen am Auge die senkrechten Säulen
31 und 41 etwa in Brusthöhe des Patienten angeordnet und
zusätzlich die Längskanten des Operationstisches 1 in
Kopfhöhe abgeschrägt sind, so daß der Operateur möglichst
nahe an den Kopf des Patienten 6 herantreten kann.

Vorstehend ist die Erfindung anhand eines Ausführungsbeispiels ohne Beschränkung des allgemeinen Erfindungsgedankens - den Laser unter dem Operationstisch anzuordnen und
den Laserstrahl durch eine im Bereich der Längskante des
Bettes angeordnete Einrichtung an die Operationsstelle zu
führen - beschrieben worden. Innerhalb des allgemeinen
Erfindungsgedankens sind selbstverständlich die verschie-

densten Modifikationen möglich. Natürlich ist der erfindungsgemäße Gedanke auch nicht auf einen bestimmten Lasertyp beschränkt, sondern kann mit beliebigen Lasern, beispielsweise Argon-Lasern zur Behandlung des Augenhintergrundes, Neodym-YAG-Lasern zur Behandlung vorderer Augenabschnitte oder mit Lasern zur radialen Keratotomie ausgeführt werden. Ferner ist es jederzeit möglich, den Erfindungsgedanken auch bei Vorrichtungen zu realisieren, die nicht zur Laserbehandlung des Auges, sondern zur Behandlung anderer Körperteile dienen. In diesem Falle kann es jedoch vorteilhaft sein, die senkrechten Säulen, in denen der Laserstrahl geführt ist, an anderen Stellen der Längskante anzuordnen, oder diese Säulen in Richtung der Längsachse verschiebbar auszubilden.

Darüberhinaus ist es auch möglich, den Schwenkarm zur Führung des Laserstrahls und den Schwenkarm für das Operationsmikroskop um die gleiche Drehachse schwenkbar auszugestalten.

An das
Europäische Patentamt

8000 München 2

| Ihr Zeichen<br>Your ref. | Ihr Schreiben vom<br>Your letter | Unser Zeichen<br>Our ref. | Datum<br>date |
|---|---|---|---|
| Neuanmeldung | | Mt 27/86 | 30.4.1987 |

Meditec Reinhardt Thyzel GmbH
8501 Heroldsberg

---

### Vorrichtung zur Laserchirurgie an einem auf einem Operationstisch liegenden Patienten

---

## A n s p r ü c h e

1. Vorrichtung zur Laserchirurgie an einem auf einem Operationstisch (1) liegenden Patienten (6) und insbesondere zur Laserbehandlung des Auges,
mit einem Laser (2), einer Strahlführungseinrichtung (3), die den Laserstrahl auf die zu behandelnde Stelle führt,
und einer Beobachtungseinrichtung (5) für die Operationsstelle,
dadurch gekennzeichnet, daß der Laser (2) einschließlich der Steuerelektronik unter dem Operationstisch (1) angeordnet ist, und daß die Strahlführungseinrichtung (3) den Laserstrahl zunächst unter dem Operationstisch (1) in Richtung der Querachse des Operationstisches führt, im Randbereich des Operationstisches (1) senkrecht nach oben und oberhalb des Operationstisches in einen horizontalen Schwenkarm (32) umlenkt, an dessen strahlaustrittsseitigem Ende eine Umlenkeinrichtung (33) den Laserstrahl auf die Operationsstelle umlenkt, und dessen Länge verstellbar ist.

2. Vorrichtung nach Anspruch 1,
dadurch gekennzeichnet, daß der Schwenkarm (32) höhenverstellbar ist.

3. Vorrichtung nach Anspruch 1 oder 2
dadurch gekennzeichnet, daß die Strahlführungseinrichtung (3) eine senkrechte Säule (31) aufweist, die an der Längsseite (11) des Operationstisches angebracht ist.

4. Vorrichtung nach Anspruch 3,
dadurch gekennzeichnet, daß die senkrechte Säule (31) der Strahlführungseinrichtung (3) für Laserbehandlungen des Auges etwa in Brusthöhe des Patienten (6) angeordnet ist.

5. Vorrichtung nach einem der Ansprüche 1 bis 4,
dadurch gekennzeichnet, daß der Schwenkarm (32) der Strahlführungseinrichtung (3) zusätzlich eine Beleuchtungseinrichtung für die Operationsstelle und insbesondere eine Spaltleuchte aufweist.

6. Vorrichtung nach einem der Ansprüche 1 bis 5,
dadurch gekennzeichnet, daß die Beobachtungseinrichtung (5) an einem weiteren Schwenkarm (42) angebracht ist, den eine senkrechte Säule (41) trägt.

7. Vorrichtung nach Anspruch 6,
dadurch gekennzeichnet, daß der weitere Schwenkarm (42)
zusätzlich um seine Längsachse (41) drehbar ist.

8. Vorrichtung nach Anspruch 6 oder 7,
dadurch gekennzeichnet, daß der weitere Schwenkarm (42)
gekröpft ist.

9. Vorrichtung nach Anspruch 8,
dadurch gekennzeichnet, daß der weitere Schwenkarm (42)
mehrfach gekröpft ist und weitere Drehgelenke aufweist.

10. Vorrichtung nach einem der Ansprüche 6 bis 9,
dadurch gekennzeichnet, daß der weitere Schwenkarm (42)
längs einer senkrechten Säule (41) höhenverstellbar ist.

11. Vorrichtung nach einem der Ansprüche 1 bis 10,
dadurch gekennzeichnet, daß die Beobachtungseinrichtung
ein Operationsmikroskop (5) ist.

12. Vorrichtung nach einem der Ansprüche 1 bis 11,
dadurch gekennzeichnet, daß die Längsseiten (11) des
Operationstisches (1) für die Laserbehandlung des Auges in
Kopfhöhe abgeschrägt sind.

Fig. 1

Fig. 2

## Europäisches Patentamt

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| Y | EP-A-0 007 256 (ARON-ROSA) <br><br> * Abbildungen; Ansprüche * <br><br> --- | 1-4,6-11 | A 61 B 17/36 <br> A 61 F 9/00 |
| Y | EP-A-0 145 892 (SIEMENS) <br><br> * Abbildung; Seite 2, Zeilen 14-17 * <br><br> --- | 1-4,6-11 | |
| A | US-A-4 520 816 (SCHACHAR) <br> * Spalte 3, Zeilen 39-43; Abbildung 1 * <br><br> --- | 5 | |
| A | FR-A-2 197 614 (KRASNOV) <br><br> ----- | | |

| RECHERCHIERTE SACHGEBIETE (Int. Cl 4) |
|---|
| A 61 B <br> A 61 F <br> A 61 G |

Der vorliegende Recherchenbericht wurde für alle Patentansprüche erstellt.

| Recherchenort | Abschlußdatum der Recherche | Prüfer |
|---|---|---|
| DEN HAAG | 26-08-1987 | STEENBAKKER J. |